# EUROPÄISCHE PATENTSCHRIFT

(11) **EP 2 826 538 B1**
(45) Veröffentlichungstag und Bekanntmachung des Hinweises auf die Patenterteilung: **16.11.2016**
(21) Anmeldenummer: 14002287.2
(22) Anmeldetag: 03.07.2014
(51) Int. Cl.: B01D 53/02, C07C 5/327, C07C 11/06, B01D 53/26

(54) **Verfahren und Vorrichtung zur Erzeugung eines Alkens**
Process and device of production of an alkene
Procédé et dispositif de production d'un alcène

(30) Priorität: 18.07.2013 DE 102013011984
(43) Veröffentlichungstag der Anmeldung: 21.01.2015
(73) Patentinhaber: Linde Aktiengesellschaft, 80331 München (DE)
(72) Erfinder: Trott, Thomas, 80686 München (DE); Klein, Bernd, 80997 München (DE)
(74) Vertreter: Fischer, Werner

(56) Entgegenhaltungen:
- WO-A1-2006/072572
- US-A- 4 520 214
- US-A- 5 227 567

## Beschreibung

Die vorliegende Erfindung betrifft ein Verfahren zur Erzeugung eines Alkens sowie eine Vorrichtung zur Durchführung des Verfahrens.

Alkene, die auch unter der Bezeichnung Olefine bekannt sind, sind chemische Verbindungen aus der Gruppe der aliphatischen Kohlenwasserstoffe, welche mindestens eine Kohlenstoff-Doppelbindung an beliebiger Position im Molekül aufweisen.

Ein bekanntes Alken ist zum Beispiel Propen, welches aus Propan durch Dehydrierung herstellbar ist. Eine derartige Dehydrierung lässt sich im sogenannten Oleflexprozess realisieren. In einem solchen Dehydrierungsprozess wird im Zuge einer kryogenen Trennung eines vom Dehydrierungs-Reaktor kommenden Gasstroms ein Wasserstoff-Volumenstrom bzw. ein wasserstoffreicher Volumenstrom sowie ein kohlenwasserstoffreiches Flüssigkeits-Endprodukt erzeugt. Im selben System wird auch ein Einsatzstoffstrom mit einem sogenannten Recycle-Gasstrom, welcher zum Beispiel ebenfalls im Wesentlichen Wasserstoff aufweist, zu einem sogenannten Kombinations-Gasstrom gemischt.

Dem aus dem Dehydrierungs-Reaktor austretenden Gas wird in einem Adsorber durch Trocknung Wasser entzogen.

Üblicherweise eingesetzte Adsorber sind zur Gewährleistung einer ausreichenden Trocknungsleistung zu regenerieren. Derartige Regenerationen führen jedoch zu Schwankungen der Temperatur, der Menge und/oder des Wasserstoffgehalts und damit auch des Molekulargewichts des dehydrierten Gases.

Das dehydrierte Gas wird in einer isolierten Umgebung, der sogenannten "Cold Box", abgekühlt und es werden die Kohlenwasserstoffe auskondensiert, die dann von der wasserstoffreichen Fraktion abgetrennt werden können. Durch die bei der Abkühlung bzw. Kondensation freigesetzte Wärme kann der zweiphasige Kombinations-Gasstrom verdampft werden und dem Dehydrierungs-Reaktor als Gasstrom eingegeben werden.

Dabei sind die physikalischen Eigenschaften des Kombinations-Gasstroms möglichst konstant zu halten. Aufgrund der Erwärmung des Kombinations-Gasstroms bzw. des Einsatzstoffes durch das den Adsorber verlassende Fluid ist, bedingt durch die Schwankungen des Betriebes des Adsorbers, nicht mehr gewährleistet, dass der Kombinations-Gasstrom konstante physikalische Parameter aufweist. Die Schwankungen im Adsorberbetrieb treten jedoch nur innerhalb einer relativ kurzen Zeitspanne bei der Adsorberregenerierung auf.

US 4,520,214 offenbart ein Verfahren zur katalytischen Dehydrierung von Alkanen. Der vorliegenden Erfindung liegt daher die Aufgabe zugrunde, ein Verfahren sowie eine Vorrichtung zur Verfügung zu stellen, mittels derer in einfacher, kostengünstiger sowie zuverlässiger Weise die Temperierung des Einsatzstoffes bzw. eines den Einsatzstoff umfassenden Volumenstromes derart gewährleistet ist, dass der in den Dehydrierungs-Reaktor gelangende Einsatzstoff bzw. der in den Dehydrierungs-Reaktor gelangende, den Einsatzstoff umfassende Volumenstrom, in seinen wesentlichen physikalischen Parametern konstant ist.

Diese Aufgabe wird durch das Verfahren zur Erzeugung eines Alkens nach Anspruch 1 sowie durch die Vorrichtung zur Erzeugung eines Alkens nach Anspruch 10 gelöst. Vorteilhafte Ausgestaltungen des erfindungsgemäßen Verfahrens sind in den Unteransprüchen 2 bis 9 angegeben. Vorteilhafte Ausgestaltungen der erfindungsgemäßen Vorrichtung sind in den Unteransprüchen 11 bis 14 angegeben.

Das erfindungsgemäße Verfahren zur Erzeugung eines Alkens, bei dem ein Kohlenwasserstoff aufweisender Einsatzstoff einer Dehydrierung unterzogen wird und ein Produktstoff, umfassend wenigstens ein Alken, erzeugt wird, umfasst erfindungsgemäß die folgenden Schritte:
- Kühlung eines aus einem Adsorber kommenden Gasstroms durch einen Gasstrom, welcher den Einsatzstoff umfasst, und Kühlung des aus dem Adsorber kommenden Gasstroms durch einen kondensierten Bestandteil des aus dem Adsorber kommenden Gasstroms in einer ersten Kühlungsphase und
- Kühlung des Einsatzstoffes in einer zweiten Kühlungsphase durch einen kondensierten Bestandteil des aus dem Adsorber kommenden Gasstroms.

Erfindungsgemäß ist vorgesehen, dass der Volumenstrom des der ersten Kühlungsphase und/ oder der zweiten Kühlungsphase zugeführten kondensierten Bestandteils des aus dem Adsorber kommenden Gasstroms in Abhängigkeit von der Temperatur des die erste Kühlungsphase verlassenden Gasstroms, der den Einsatzstoff umfasst und der der Dehydrierung zugeführt wird, variiert wird.

Insbesondere ist dieses Verfahren zur Erzeugung eines Alkens bzw. Olefins wie z.B. Propen bzw. eines Propan-Propen-Gemischs anwendbar. Ein solches Alken bzw. ein solches Gemisch wird nach der beschriebenen Herstellung anschließend einem sogenannten C3-Splitter zugeführt.

Der aus dem Adsorber kommende Gasstrom ist ein sogenannter "Cold Box Vapor Feed", d. h., ein den Einsatzstoff umfassender Gas-Volumenstrom, der zuvor den Dehydrierungs-Reaktor zwecks Wasserstoffabscheidung sowie einen Adsorber zur Entfernung von Wasser bzw. zur Trocknung des aus dem Dehydrierungs-Reaktor kommenden Fluides durchströmt hat.

Weiterhin ist vorteilhaft vorgesehen, dass der Einsatzstoffstrom mit einem Recycle-Gasstrom zu einem Kombinations-Gasstrom gemischt wird. Der Recycle-Gasstrom ist vorzugsweise Wasserstoff oder ein im Wesentlichen Wasserstoff enthaltendes Gasgemisch, welcher bzw. welches als Nebenprodukt zum Produktstoff entsteht.

Das erfindungsgemäße Verfahren ist insbesondere dann vorteilhaft ausgebildet, wenn in der zweiten Kühlungsphase die Kühlung des Einsatzstoffes durch Übertragung von Kälte des kondensierten Bestandteils des aus dem Adsorber kommenden Gasstroms auf einen Einsatzstoffstrom erfolgt.

Zwecks Kühlung ist dabei zur einfachen Ausgestaltung des Verfahrens Übertragung von Kälte des kondensierten Bestandteils des aus dem Adsorber kommenden Gasstroms auf den aus dem Adsorber kommenden Gasstrom in einem ersten Wärmetauscher vorgesehen. Die Übertragung von Kälte des kondensierten Bestandteils des aus dem Adsorber kommenden Gasstroms auf den Einsatzstoffstrom erfolgt dabei in einem zweiten Wärmetauscher.

Die Übertragung von Kälte vom kondensierten Bestandteil des aus dem Adsorber kommenden Gasstroms auf den aus dem Adsorber kommenden Gasstrom und/ oder die Übertragung von Kälte vom Gasstrom, welcher den Einsatzstoff umfasst, auf den

aus dem Adsorber kommenden Gasstrom wird in Abhängigkeit von der Temperatur des die erste Kühlungsphase verlassenden Gasstroms, der den Einsatzstoff umfasst und der der Dehydrierung zugeführt wird, durchgeführt. Das Verfahren zur Erzeugung eines Alkens kann dabei derart ausgeführt sein, dass die erste Kühlungsphase und die zweite Kühlungsphase zumindest zeitabschnittsweise gleichzeitig verlaufen.

Zwecks Steuerung der Kühlung ist vorzugsweise vorgesehen, dass stromabwärts des Adsorbers wenigstens ein Parameter des Stoffes oder des Volumenstroms des aus den Adsorber kommenden Gasstroms bestimmt wird, dieser Parameter mit einem zulässigen Grenzwertbereich verglichen wird und bei Abweichung des bestimmten Parameters vom Grenzwertbereich wenigstens ein Parameter des Stoffes und/oder des Volumenstromes des kondensierten Bestandteils des aus dem Adsorber kommenden Gasstroms variiert wird. Die Bestimmung des Parameters des Stoffes oder des Volumenstromes des aus dem Adsorber kommenden Gasstroms erfolgt vorzugsweise im ersten Wärmetauscher. Ein zu bestimmender Parameter kann insbesondere ein physikalischer Parameter wie z. B. die Temperatur oder die Geschwindigkeit des Volumenstroms sein. Der Grenzwertbereich, mit dem der Parameter verglichen wird, ist ein Parameter-Grenzwertbereich, der je nach Kühlungsleistungsanforderung definiert und vorzugsweise abrufbar gespeichert ist. Dadurch lassen sich in einfacher Weise etwaige vom Adsorber bewirkte Schwankungen hinsichtlich der Temperatur, der Masse, des Volumenstroms und/oder des Wasserstoffgehalts des aus dem Adsorber kommenden Gasstroms und dessen daraus resultierender Kühlungsleistung ausgleichen.

Vorteilhafterweise ist vorgesehen, dass der kondensierte Bestandteil des aus dem Adsorber kommenden Gasstroms in einer Speichereinrichtung gespeichert wird und von dort je nach Kühlungsleistungsanforderung der ersten und/ oder zweiten Kühlungsphase zugeführt wird. Das heißt, dass die Zuführung aus der Speichereinrichtung in Abhängigkeit von der Temperatur des die erste Kühlungsphase verlassenden Gasstroms, der den Einsatzstoff umfasst und der der Dehydrierung zugeführt wird, erfolgt.

In bevorzugter Ausgestaltung des erfindungsgemäßen Verfahrens ist vorgesehen, dass die zweite Kühlungsphase stromaufwärts der ersten Kühlungsphase durchgeführt wird. Diese Position bezieht sich auf den Volumenstrom des Einsatzstoffes. In weiterer vorteilhafter Ausgestaltung des erfindungsgemäßen Verfahrens ist vorgesehen, dass als ein weiterer Produktstoff Wasserstoff erhalten wird und der erhaltene Wasserstoff zumindest anteilig zur Stabilisierung des Wasserstoff-Abspaltungsprozesses in einen Reaktor zur Wasserstoffabspaltung geleitet wird.

Der Wasserstoff wird vorzugsweise durch Auftrennung des Stoffgemisches erhalten.

Zur Lösung der genannten Aufgabe wird außerdem erfindungsgemäß eine Vorrichtung zur Erzeugung eines Alkens zur Verfügung gestellt, die zur Dehydrierung eines ein Kohlenwasserstoff aufweisenden Einsatzstoffes und zur Erzeugung eines Produktstoffes, der wenigsten ein Alken umfasst, eingesetzt wird. Diese Vorrichtung umfasst einen Adsorber zur Adsorption von Wasser aus einem Kombinations-Gasstrom, der den Einsatzstoff mit einem Gasstrom, welcher den Einsatzstoff umfasst, kombiniert; eine erste Kühlungseinrichtung zur Kühlung eines aus einem Adsorber kommenden Gasstroms durch den Gasstrom, welcher den Einsatzstoff umfasst, und zur Kühlung des aus dem Adsorber kommenden Gasstroms durch einen kondensierten Bestandteil des aus dem Adsorber kommenden Gasstroms in einer ersten Kühlungsphase; eine Speichereinrichtung zur Speicherung eines kondensierten Bestandteils des aus dem Adsorber kommenden Gasstroms und eine zweite Kühlungseinrichtung zur Kühlung des Einsatzstoffes in einer zweiten Kühlungsphase durch den kondensierten Bestandteil des aus dem Adsorber kommenden Gasstroms. Erfindungsgemäß weist die Vorrichtung eine Steuer- und/oder Regelungseinrichtung auf, mit der automatisiert der Volumenstrom des aus der Speichereinrichtung der ersten und/ oder zweiten Kühlungsphase zuzuführenden kondensierten Bestandteils des aus dem Adsorber kommenden Gasstroms in Abhängigkeit von der Temperatur des die erste Kühlungsphase verlassenden Gasstroms, der den Einsatzstoff umfasst und der der Dehydrierung zugeführt wird, variierbar ist.

Die erfindungsgemäße Vorrichtung dient zur Dehydrierung eines Kohlenwasserstoffs bzw. zur Durchführung des erfindungsgemäßen Verfahrens.

Der Adsorber wird zur Trocknung des den Adsorber durchströmenden Mediums eingesetzt.

Weiterhin umfasst die erfindungsgemäße Vorrichtung vorzugsweise noch wenigstens eine und bevorzugt mehrere Kondensationseinrichtungen bzw. Abscheider zur Abtrennung des kondensierten Bestandteils des aus dem Adsorber kommenden Gasstroms.

Zur Aufnahme des Kondensats sollte der jeweilige Abscheider ein Gefäß aufweisen.

Das Speichervolumen der Speichereinrichtung ist derart bemessen, dass die Speichereinrichtung ausreichend Kondensat zum Ausgleich der Kälteübertragungsschwankung in der ersten Kühlungseinrichtung zur Verfügung stellen kann. Die Kühlungseinrichtungen sind vorzugsweise Wärmetauscher, wobei die Übertragung von Wärme sich auf das wärmeabgebende Medium kühlend auswirkt.

Das heißt, dass die Steuer- und/oder Regelungseinrichtung derart ausgestaltet ist, dass mit ihr automatisch der Volumenstrom einstellbar ist, der in Abhängigkeit von der Temperatur des die erste Kühlungsphase verlassenden Gasstroms, der den Einsatzstoff umfasst und der der Dehydrierung zugeführt wird, in der ersten und der zweiten Kühlungsphase benötigt wird, um die Schwankungen des Adsorberbetriebes und demzufolge die Schwankungen des aus dem Adsorber kommenden Gasstroms, die wiederum Temperierungsschwankungen in der ersten Kühlungsphase bewirken, auszugleichen.

Die Speichereinrichtung kann einen Behälter und/oder einen Abscheider zur Aufnahme des kondensierten Bestandteils des aus dem Adsorber kommenden Gasstroms umfassen.

In einer wärmetechnisch effizienten Ausgestaltung der erfindungsgemäßen Vorrichtung ist vorgesehen, dass diese einen wärmetechnisch isolierten Raum umfasst, in dem die Kühlungseinrichtungen angeordnet sind, wobei der Behälter der Speichereinrichtung außerhalb dieses wärmetechnisch isolierten Raumes angeordnet ist. Ein derartiger wärmetechnisch isolierter Raum ist eine sogenannte "Cold Box" zur Minimierung der Temperatur der sie durchströmenden Fluide. Aufgrund der moderaten Temperatur im Behälter der Speichereinrichtung besteht keine Notwendigkeit, diesen ebenfalls im wärmetechnisch isolierten Raum anzuordnen. Die Anordnung des Behälters außerhalb des wärmetechnisch isolierten Raumes hat zudem den Vorteil, dass der wärmetechnisch isolierte Raum nicht mit einem entsprechenden Volumen zur Aufnahme des relativ großen Behälters ausgestaltet sein braucht. Aus Gründen der Energieeffizienz soll aber nicht ausgeschlossen werden, dass der Behälter der Speichereinrichtung ebenfalls im wärmetechnisch isolierten Raum angeordnet wird.

Zur Beeinflussung des Volumenstromes in die erste und/ oder zweite Kühlungsphase umfasst die Vorrichtung im Strömungspfad des kondensierten Bestandteils des aus dem Adsorber kommenden Gasstroms eine Pumpe zur Förderung des Kondensats in wenigstens eine Kühlungseinrichtung. Vorzugsweise ist diese Pumpe stromabwärts des Behälters der Speichereinrichtung und somit auch außerhalb des wärmetechnisch isolierten Raums angeordnet.

Außerdem sollte stromabwärts des Adsorbers oder auch als ein Bestandteil des Adsorbers ein Verdichter zur Verdichtung des aus dem Adsorber kommenden Gasstromes angeordnet sein.

In weiterer günstiger Ausgestaltung der erfindungsgemäßen Vorrichtung weist wenigstens eine der Kühlungseinrichtungen eine Mischeinrichtung zur Mischung eines Recycle-Gasstroms mit dem Einsatzstoff zu einem Kombinations-Gasstrom auf. Der Recycle-Gasstrom ist ein Volumenstrom aus Wasserstoff oder einem im Wesentlichen Wasserstoff enthaltenden Gas, welches als Nebenprodukt zum Produktstoff entsteht.

Der Vorteil des erfindungsgemäßen Verfahrens sowie der erfindungsgemäßen Vorrichtung liegt insbesondere darin, dass Betriebsschwankungen des Adsorbers durch Zuführung einer geeigneten Menge bzw. eines geeignet dimensionierten Volumenstroms des Kondensats des aus dem Adsorber kommenden Gases aus einem dem Adsorber nachgeschalteten Speicherbehälter in die erste und/ oder zweite Kühlungsphase kompensiert werden können. Dadurch wird gewährleistet, dass die physikalischen Parameter des der Dehydrierung zuzuführenden Gasstromes konstant gehalten werden können. Das heißt, dass selbst bei Temperaturschwankungen des den Adsorber verlassenden Fluides aufgrund der Einstellung des der ersten und/ oder zweiten Kühlungsphase zuzuführenden Volumenstromes die herkömmlich verwendeten und dimensionierten Wärmetauscher weiterhin verwendbar sind. Es besteht somit nicht die Notwendigkeit, aufgrund der in den Wärmetauschern gegebenenfalls sich einstellenden geringeren Temperaturdifferenz größere Wärmetauscher anzuordnen, die hinsichtlich ihrer Herstellungs- und/oder Betriebskosten aufwändig sind.

Weitere Einzelheiten und Vorteile der Erfindung sollen durch die nachfolgende Figurenbeschreibung eines in der Figur dargestellten Ausführungsbeispiels erläutert werden.

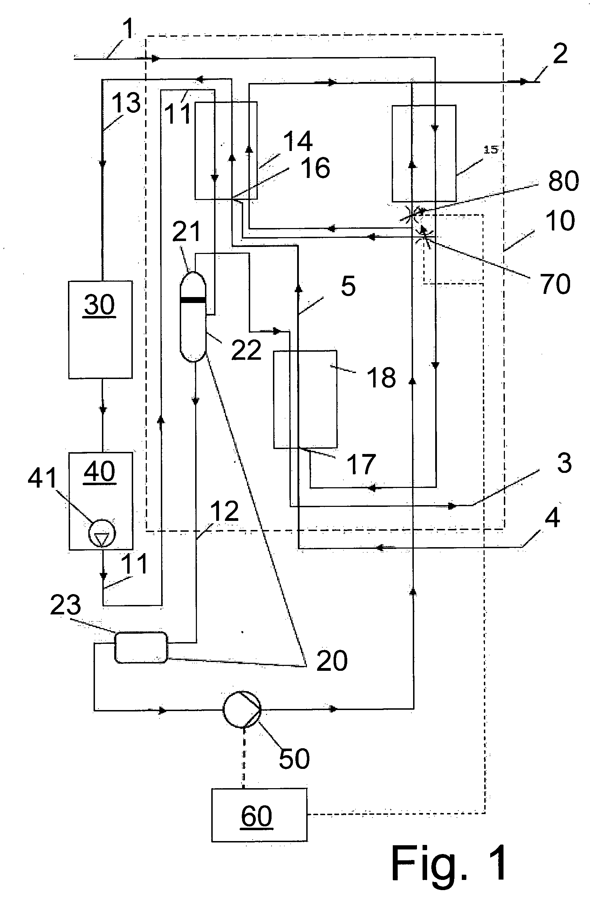

Figur 1 zeigt eine erfindungsgemäße Vorrichtung in schematischer Darstellung.

Aus Figur 1 ist ersichtlich, dass ein Einsatzstoff 1, der z. B. Propan sein kann, einem System bzw. der dargestellten Vorrichtung zugeführt wird. Dieser Einsatzstoff 1 durchströmt eine zweite Kühlungseinrichtung 15, die vorzugsweise als Wärmetauscher ausgestaltet ist. Nach der dargestellten Verzweigung durchströmt der Einsatzstoff 1 einerseits eine erste Kühlungseinrichtung 14 und wird andererseits mit einem Recycle-Gasstrom 4, der z. B. im Wesentlichen Wasserstoff umfassen kann, in einer zweiten Mischeinrichtung 17, die bevorzugt als ein Wärmetauscher ausgestaltet ist, gemischt. Dieses Gemisch wird dabei in der zweiten Mischeinrichtung 17 verdampft und als ein Gasstrom 5, welcher den Einsatzstoff umfasst, zur ersten Kühlungseinrichtung 14 weitergeführt. Vor Eintritt in die erste Kühlungseinrichtung 14 werden der Einsatzstoff 1 sowie der Gasstrom 5, welcher den Einsatzstoff umfasst, in einer ersten Mischeinrichtung 16 zu einem Kombinations-Gasstrom 13 vermischt. Dabei wird dieses Gemisch wieder verdampft. Der erzeugte Kombinations-Gasstrom 13 durchströmt den Reaktor 30, in dem dieser Gasstrom dehydriert wird. Das dehydrierte Gas wird danach durch den Adsorber 40 geleitet, in dem es getrocknet wird und anschließend im Verdichter 41 verdichtet wird. Der aus dem Adsorber kommende Gasstrom 11 wird wiederum durch die erste Kühlungseinrichtung 14 geleitet, wo er Wärme auf den Kombinations-Gasstrom 13 abgibt und somit dieses Fluid erwärmt. Der aus dem Adsorber kommende Gasstrom 11 gelangt dann in einen Abscheider 21 und dessen Gefäß 22, von dem aus der kondensierte Bestandteil des aus dem Adsorber kommenden Gasstroms 12 in einen Behälter 23 geleitet wird. Das in dem Abscheider 21 abgetrennte Gas wird als weiterer Stoff 3, der im Wesentlichen Wasserstoff umfasst, abgeleitet. Im Strömungspfad des kondensierten Bestandteils des aus dem Adsorber kommenden Gasstroms 12 ist stromabwärts des Behälters 23 eine Pumpe 50 angeordnet, die mit einer Steuer- und/oder Regelungseinrichtung 60 bedienbar ist. Diese Pumpe 50 fördert den kondensierten Bestandteil des aus dem Adsorber kommenden Gasstroms 12 durch die erste Kühlungseinrichtung 14 sowie durch die zweite Kühlungseinrichtung 15, wobei die Kälte dieses Mediums auf den aus dem Adsorber 40 kommenden Gasstrom in der ersten Kühlungseinrichtung sowie auf den die zweite Kühlungseinrichtung 15 durchströmenden Einsatzstoff 1 abgegeben wird. Danach verlässt dieses Fluid als Produktstoff 2 das System.

Weiterhin sind zwei Stromventile 70, 80 angeordnet, mit denen jeweils der Volumenstrom des kondensierten Bestandteils des aus dem Adsorber kommenden Gasstroms 12 zur ersten Kühlungseinrichtung 14 und zur zweiten Kühlungseinrichtung 15 einstellbar ist, wobei die Steuer- und/oder Regelungseinrichtung 60 mit der Pumpe 50 und / oder den Stromventilen 70, 80 steuerungstechnisch verbunden ist. Insbesondere können die Stromventile 70,80 einstellbare Drosselventile sein.

Das den Abscheider 21 verlassende Gas wird unter Wärmeabgabe in einer dritten Kühlungseinrichtung 18 zwecks Wärmeübertragung auf den durch diese Kühleinrichtung geleiteten Einsatzstoff 1 gekühlt.

In der dargestellten Ausführungsvariante der erfindungsgemäßen Vorrichtung sind alle drei Kühlungseinrichtungen 14, 15, 18 sowie der Abscheider 21 und die Mischeinrichtungen 16, 17 in einem wärmetechnisch isolierten Raum angeordnet.

Der Abscheider 21 bzw. dessen an seiner Unterseite angeordnetes Gefäß 22 und der Behälter 23 bilden eine Speichereinrichtung 20 aus, die zur Zwischenspeicherung des kondensierten Bestandteils des aus dem Adsorber kommenden Gasstroms 12 ausgebildet ist.

Beim Betrieb des Adsorbers 40 bzw. bei dessen Regenerierung können Leistungsschwankungen auftreten, so dass die Temperatur und/oder das Volumen bzw. die Geschwindigkeit des Volumenstromes des aus dem Adsorber kommenden Gasstroms 11 derart variiert, dass der wärmetechnisch isolierte Raum, die sogenannten "Cold Box" nicht mehr genügend Kondensat des aus dem Adsorber 40 kommenden Gasstroms erzeugen kann, so dass die Gefahr besteht, dass der Kombinations-Gasstrom 13 nicht die erforderliche Reinheit aufweist und somit die Dehydrierung im Reaktor 30 nicht mit dem gewünschten Ergebnis realisierbar ist.

Um dies zu vermeiden, werden die Pumpe 50 und/ oder die Stromventile 70, 80 durch die Steuer- und/oder Regelungseinrichtung 60 derart betrieben bzw. angesteuert, dass bei festgestellter Abweichung eines Parameters, insbesondere eines physikalischen Parameters, des aus dem Adsorber kommenden Gasstroms 11 von einem vorher definierten Grenzwertbereich aus dem Behälter 23 und/oder aus dem Gefäß 22 des Abscheiders 21 mehr kondensierter Bestandteil des aus dem Adsorber kommenden Gasstroms 12 entnommen wird und der ersten Kühlungseinrichtung 14 und/ oder der zweiten Kühlungseinrichtung 15 zugeführt wird, als im Normalbetrieb des Adsorbers 40 nötig ist. Dadurch wird erreicht, dass trotz Leistungsschwankungen des Adsorbers 40 eine ausreichende Kühlungswirkung in der ersten Kühlungseinrichtung 14 sowie der zweiten Kühlungseinrichtung 15 realisiert wird und demzufolge keine weiteren Maßnahmen zur ausreichenden Kühlung des Kombinations-Gasstromes 13 ergriffen werden müssen wie z. B. ein Einsatz größerer Wärmetauscher und/oder Zuführung von Fremdkälte.

**Bezugszeichenliste**

| | |
|---|---|
| Einsatzstoff | 1 |
| Produktstoff | 2 |
| weiterer Stoff | 3 |
| Recycle-Gasstrom | 4 |
| Einsatzstoff umfassender Gasstrom | 5 |
| wärmetechnisch isolierter Raum | 10 |
| aus dem Adsorber kommender Gasstrom | 11 |
| kondensierter Bestandteil des aus dem Adsorber kommenden Gasstroms | 12 |
| Kombinations-Gasstrom | 13 |
| erste Kühlungseinrichtung | 14 |
| zweite Kühlungseinrichtung | 15 |
| erste Mischeinrichtung | 16 |
| zweite Mischeinrichtung | 17 |
| dritte Kühlungseinrichtung | 18 |
| Speichereinrichtung | 20 |
| Abscheider | 21 |
| Gefäß | 22 |
| Behälter | 23 |
| Reaktor | 30 |
| Adsorber | 40 |
| Verdichter | 41 |
| Pumpe | 50 |
| Steuer- und/ oder Regelungseinrichtung | 60 |
| erstes Stromventil | 70 |
| zweites Stromventil | 80 |

## Patentansprüche

1. Verfahren zur Erzeugung eines Alkens, bei dem ein Kohlenwasserstoff aufweisender Einsatzstoff (1) einer Dehydrierung in einem Dehydrierungsreaktor (30) unterzogen wird und ein Produktstoff (2), umfassend wenigstens ein Alken, erzeugt wird, mit den folgenden Schritten:
- Kühlung eines aus einem stromabwärts des Dehydrierungsreaktors (30) zur Adsorption von Wasser angeordneten Adsorber (40) kommenden Gasstroms (11) durch einen Gasstrom (5), welcher den Einsatzstoff umfasst, und durch einen kondensierten Bestandteil (12) des aus dem Adsorber (40) kommenden Gasstroms in einer ersten Kühlungsphase und
- Kühlung des Einsatzstoffes (1) in einer zweiten Kühlungsphase durch einen kondensierten Bestandteil (12) des aus dem Adsorber (40) kommenden Gasstroms, **dadurch gekennzeichnet,**
**dass** der Volumenstrom des der ersten Kühlungsphase und/ oder der zweiten Kühlungsphase zugeführten kondensierten Bestandteils (12) des aus dem Adsorber (40) kommenden Gasstroms in Abhängigkeit von der Temperatur des die erste Kühlungsphase verlassenden Gasstromes (13), der den Einsatzstoff umfasst und der dem Dehydrierungsreaktor (30) zugeführt wird, variiert wird.

2. Verfahren zur Erzeugung eines Alkens nach Anspruch 1, **dadurch gekennzeichnet, dass** der Einsatzstoff (1) als Einsatzstoffstrom vorliegt und dieser mit einem Recycle-Gasstrom (4) zu einem Kombinations-Gasstrom (13) gemischt wird.

3. Verfahren zur Erzeugung eines Alkens nach Anspruch 2, **dadurch gekennzeichnet, dass** in der zweiten Kühlungsphase die Kühlung des Einsatzstoffes (1) durch Übertragung von Kälte des kondensierten Bestandteils (12) des aus dem Adsorber (40) kommenden Gasstroms auf einen Einsatzstoffstrom erfolgt.

4. Verfahren zur Erzeugung eines Alkens nach Anspruch 3, **dadurch gekennzeichnet, dass** Übertragung von Kälte des Gasstromes (5), welcher den Einsatzstoff umfasst, auf den aus dem Adsorber kommenden Gasstrom (11), und Übertragung von Kälte des kondensierten Bestandteils (12) des aus dem Adsorber (40) kommenden Gasstroms auf den aus dem Adsorber (40) kommenden Gasstrom (11) in einem ersten Wärmetauscher erfolgt, und die Übertragung von Kälte des kondensierten Bestandteils (12) des aus dem Adsorber (40) kommenden Gasstroms auf den Einsatzstoffstrom in einem zweiten Wärmetauscher erfolgt.

5. Verfahren zur Erzeugung eines Alkens nach einem der vorhergehenden Ansprüche, **dadurch gekennzeichnet, dass** die erste und die zweite Kühlungsphase zumindest zeitabschnittsweise gleichzeitig verlaufen.

6. Verfahren zur Erzeugung eines Alkens nach einem der vorhergehenden Ansprüche, **dadurch gekennzeichnet, dass** stromabwärts des Adsorbers (40) wenigstens ein Parameter des Stoffes oder des Volumenstromes des aus dem Adsorber (40) kommenden Gasstroms (11) bestimmt wird, dieser Parameter mit einem zulässigen Grenzwertbereich verglichen wird und bei Abweichung des bestimmten Parameters vom Grenzwertbereich wenigstens ein Parameter des Stoffes und/ oder des Volumenstroms des kondensierten Bestandteils (12) des aus dem Adsorber kommenden Gasstroms variiert wird.

7. Verfahren zur Erzeugung eines Alkens nach einem der vorhergehenden Ansprüche, **dadurch gekennzeichnet, dass** der kondensierte Bestandteil (12) des aus dem Adsorber (40) kommenden Gasstroms in einer Speichereinrichtung (20) gespeichert wird und von dort je nach Kühlungsleistungsanforderung der ersten und/ oder zweiten Kühlungsphase zugeführt wird.

8. Verfahren zur Erzeugung eines Alkens nach einem der vorhergehenden Ansprüche, **dadurch gekennzeichnet, dass** die zweite Kühlungsphase stromaufwärts der ersten Kühlungsphase durchgeführt wird.

9. Verfahren zur Erzeugung eines Alkens nach einem der vorhergehenden Ansprüche, **dadurch gekennzeichnet, dass** als ein weiterer Produktstoff (3) Wasserstoff erhalten wird und der erzeugte Wasserstoff zumindest anteilig zur Stabilisierung des Wasserstoff-Abspaltungsprozesses in einen Reaktor (30) zur Wasserstoffabspaltung geleitet wird.

10. Vorrichtung zur Erzeugung eines Alkens, bei der ein einen Kohlenwasserstoff aufweisender Einsatzstoff (1) einer Dehydrierung unterzogen wird und ein Produktstoff (2), umfassend wenigstens ein Alken, erzeugt wird, umfassend:
- einen Dehydrierungsreaktor (30) zur Erzeugung des Alkens aus dem Kohlenwasserstoff,
- einen stromabwärts des Dehydrierungsreaktors (30) angeordneten Adsorber (40) zur Adsorption von Wasser aus dem im Dehydrierungsreaktor (30) erhaltenen Stoffstrom,
- eine erste Kühlungseinrichtung (14) zur Kühlung eines aus einem Adsorber (40) kommenden Gasstroms (11) durch den Gasstrom (5), welcher den Einsatzstoff umfasst, und durch einen kondensierten Bestandteil (12) des aus dem Adsorber (40) kommenden Gasstroms in einer ersten Kühlungsphase,
- eine Speichereinrichtung (20) zur Speicherung eines kondensierten Bestandteils (12) des aus dem Adsorber (40) kommenden Gasstroms,
- eine zweite Kühlungseinrichtung (15) zur Kühlung des Einsatzstoffes (1) in einer zweiten Kühlungsphase durch den kondensierten Bestandteil (12) des aus dem Adsorber (40) kommenden Gasstroms, **dadurch gekennzeichnet,**
**dass** die Vorrichtung eine Steuer- und/ oder Regelungseinrichtung (60) aufweist, mit der automatisiert der Volumenstrom des aus der Speichereinrichtung (20) der ersten und/ oder zweiten Kühlungsphase zuzuführenden kondensierten Bestandteils (12) des aus dem Adsorber (40) kommenden Gasstroms in Abhängigkeit von der Temperatur des die erste Kühlungsphase verlassenden Gasstromes (13), der den Einsatzstoff umfasst und der der Dehydrierung zugeführt wird, variierbar ist.

11. Vorrichtung zur Erzeugung eines Alkens nach Anspruch 10, **dadurch gekennzeichnet, dass** die Speichereinrichtung (20) einen Behälter (23) und/ oder wenigstens einen Abscheider (21) zur Abtrennung des kondensierten Bestandteils (12) des aus dem Adsorber (40) kommenden Gasstroms umfasst.

12. Vorrichtung zur Erzeugung eines Alkens nach Anspruch 11, **dadurch gekennzeichnet, dass** die Vorrichtung einen wärmetechnisch isolierten Raum (10) umfasst, in dem die Kühlungseinrichtungen (14, 15, 18) angeordnet sind, wobei der Behälter (23) außerhalb dieses wärmetechnisch isolierten Raumes (10) angeordnet ist.

13. Vorrichtung zur Erzeugung eines Alkens nach einem der Ansprüche 10 bis 12, **dadurch gekennzeichnet, dass** im Strömungspfad des kondensierten Bestandteils des aus dem Adsorber (40) kommenden Gasstroms (12) eine Pumpe (50) zur Förderung des kondensierten Bestandteils des aus dem Adsorber kommenden Gasstroms (12) in wenigstens eine Kühlungseinrichtung (14, 15, 18) angeordnet ist.

14. Vorrichtung zur Erzeugung eines Alkens nach einem der Ansprüche 10 bis 13, **dadurch gekennzeichnet, dass** wenigstens eine der Kühlungseinrichtungen (14, 15, 18) eine Mischeinrichtung (16, 17) zur Mischung eines Recycle-Gasstroms (4) mit dem Einsatzstoff (1) zu einem Kombinations-Gasstrom (13) aufweist.

## Claims

1. Method for generating an alkene, in which a hydrocarbon-comprising feed material (1) is subjected to a dehydrogenation in a dehydrogenation reactor (30) and a product material (2) comprising at least one alkene is generated, having the following steps:
- cooling a gas stream (11) coming from an adsorber (40) arranged downstream of the dehydrogenation reactor (30) for adsorption of water by a gas stream (5) which comprises the feed material, and by a condensed component (12) of the gas stream coming from the adsorber (40) in a first cooling phase and
- cooling the feed material (1) in a second cooling phase by a condensed component (12) of the gas stream coming from the adsorber (40),
**characterized in that**
the volumetric stream of the condensed component (12) of the gas stream coming from the adsorber (40), which condensed component is fed to the first cooling phase and/or the second cooling phase, is varied in dependence on the temperature of the gas stream (13) leaving the first cooling phase which comprises the feed material and which is fed to the dehydrogenation reactor (30).

2. Method for generating an alkene according to Claim 1, **characterized in that** the feed material (1) is present as feed material stream and this is mixed with a recycled gas stream (4) to form a combined gas stream (13).

3. Method for generating an alkene according to Claim 2, **characterized in that,** in the second cooling phase, the feed material (1) is cooled by transfer of cold from the condensed component (12) of the gas stream coming from the adsorber (40) to a feed material stream.

4. Method for generating an alkene according to Claim 3, **characterized in that,** transfer of cold from the gas stream (5) which comprises the feed material to the gas stream (11) coming from the adsorber and transfer of cold from the condensed component (12) of the gas stream coming from the adsorber (40) to the gas stream (11) coming from the adsorber (40) proceeds in a first heat exchanger, and the transfer of cold from the condensed component (12) of the gas stream coming from the adsorber (40) to the feed material stream proceeds in a second heat exchanger.

5. Method for generating an alkene according to any one of the preceding claims, **characterized in that** the first cooling phase and the second cooling phase run simultaneously at least for periods of time.

6. Method for generating an alkene according to any one of the preceding claims, **characterized in that,** downstream of the adsorber (40), at least one parameter of the material or of the volumetric stream of the gas stream (11) coming from the adsorber (40) is determined, this parameter is compared with a permissible limiting value range and, in the event of deviation of the determined parameter from the limiting value range, at least one parameter of the material and/or of the volumetric stream of the condensed component (12) of the gas stream coming from the adsorber is varied.

7. Method for generating an alkene according to any one of the preceding claims, **characterized in that** the condensed component (12) of the gas stream coming from the adsorber (40) is stored in a storage appliance (20) and from there is fed to the first and/or second cooling phase according to refrigeration performance requirement.

8. Method for generating an alkene according to any one of the preceding claims, **characterized in that** the second cooling phase is carried out upstream of the first cooling phase.

9. Method for generating an alkene according to any one of the preceding claims, **characterized in that** hydrogen is obtained as a further product material (3) and the hydrogen generated is at least partially led into a reactor (30) for hydrogen elimination for stabilization of the hydrogen elimination process.

10. Device for generating an alkene, in which a hydrocarbon-comprising feed material (1) is subjected to a dehydrogenation, and a product material (2) comprising at least one alkene is generated, comprising:
- a dehydrogenation reactor (30) for generating the alkene from the hydrocarbon,
- an adsorber (40) arranged downstream of the dehydrogenation reactor (30) for adsorption of water from the material stream obtained in the dehydrogenation reactor (30),
- a first cooling appliance (14) for cooling a gas stream (11) coming from an adsorber (40) by the gas stream (5) which comprises the feed material, and by a condensed component (12) of the gas stream coming from the adsorber (40) in a first cooling phase,
- a storage appliance (20) for storage of a condensed component (12) of the gas stream coming from the adsorber (40),
- a second cooling appliance (15) for cooling the feed material (1) in a second cooling phase by the condensed component (12) of the gas stream coming from the adsorber (40),
**characterized in that**
the device has an open-loop and/or closed-loop control appliance (60), with which the volumetric stream of the condensed component (12) of the gas stream coming from the adsorber (40), which condensed component is to be fed from the storage appliance (20) to the first and/or second cooling phase, is variable in an automated manner in dependence on the temperature of the gas stream (13) that is leaving the first cooling phase, comprises the feed material and is fed to the dehydrogenation.

11. Device for generating an alkene according to Claim 10, **characterized in that** the storage appliance (20) comprises a container (23) and/or at least one separator (21) for separating off the condensed component (12) of the gas stream coming from the adsorber (40).

12. Device for generating an alkene according to Claim 11, **characterized in that** the device comprises a heat-insulated space (10) in which the cooling appliances (14, 15, 18) are arranged, wherein the container (23) is arranged outside this heat-insulated space (10).

13. Device for generating an alkene according to any one of Claims 10 to 12, **characterized in that** a pump (50) for transporting the condensed component of the gas stream (12) coming from the adsorber into at least one cooling appliance (14, 15, 18) is arranged in the flow path of the condensed component of the gas stream (12) coming from the adsorber (40).

14. Device for generating an alkene according to any one of Claims 10 to 13, **characterized in that** at least one of the cooling appliances (14, 15, 18) has a mixing appliance (16, 17) for mixing a recycled gas stream (4) with the feed material (1) to form a combined gas stream (13).

## Revendications

1. Procédé de production d'un alcène, dans lequel on soumet une substance de départ (1), comprenant un hydrocarbure, à une déshydrogénation dans un réacteur de déshydrogénation (30), et on produit un produit (2) comprenant au moins un alcène, comportant les étapes suivantes :
- refroidissement, dans une première phase de refroidissement, d'un courant gazeux (11), provenant d'un adsorbeur (40) disposé en aval du réacteur de déshydrogénation (30) et destiné à l'adsorption de l'eau, grâce à un courant gazeux (5) comprenant la substance de départ et à un constituant condensé (12) du courant gazeux provenant de l'adsorbeur (40), et
- refroidissement de la substance de départ (1) dans une deuxième phase de refroidissement, grâce à un constituant condensé (12) du courant gazeux provenant de l'adsorbeur (40),
**caractérisé en ce qu'**on fait varier le débit volumique du constituant condensé (12), amené à la première phase de refroidissement et/ou à la deuxième phase de refroidissement, du courant gazeux provenant de l'adsorbeur (40), en fonction de la température du courant gazeux (13) sortant de la première phase de refroidissement, qui comprend la substance de départ et est envoyé au réacteur de déshydrogénation (30).

2. Procédé de production d'un alcène selon la revendication 1, **caractérisé en ce que** la substance de départ (1) se présente sous forme d'un courant de substance de départ, et que ce dernier est mélangé à un courant gazeux de recyclage (4) pour obtenir un courant gazeux combiné (13).

3. Procédé de production d'un alcène selon la revendication 2, **caractérisé en ce que**, dans la deuxième phase de refroidissement, le refroidissement de la substance de départ (1) est réalisé par transfert, sur un courant de substance de départ, du froid du constituant condensé (12) du courant gazeux provenant de l'adsorbeur (40).

4. Procédé de production d'un alcène selon la revendication 3, **caractérisé en ce que** le transfert du froid du courant gazeux (5), qui comprend la substance de départ, au courant gazeux (11) provenant de l'adsorbeur, et le transfert du froid du constituant condensé (12) du courant gazeux provenant de l'adsorbeur (40) au courant gazeux (11) provenant de l'adsorbeur (40), ont lieu dans un premier échangeur de chaleur, et le transfert du froid du constituant condensé (12) du courant gazeux provenant de l'adsorbeur (40) au courant de substance de départ a lieu dans un deuxième échangeur de chaleur.

5. Procédé de production d'un alcène selon l'une des revendications précédentes, **caractérisé en ce que** la première et la deuxième phases de refroidissement se déroulent simultanément au moins par séquences temporelles.

6. Procédé de production d'un alcène selon l'une des revendications précédentes, **caractérisé en ce que**, en aval de l'adsorbeur (40), on détermine au moins un paramètre de la substance ou du débit volumique du courant gazeux (11) provenant de l'adsorbeur (40), on compare ce paramètre à une plage autorisée de valeurs limites et, en cas d'écart entre le paramètre déterminé et la plage de valeurs limites, on fait varier au moins un paramètre de la substance et/ou du débit volumique du constituant condensé (12) du courant gazeux provenant de l'adsorbeur.

7. Procédé de production d'un alcène selon l'une des revendications précédentes, **caractérisé en ce que** le constituant condensé (12) du courant gazeux provenant de l'adsorbeur (40) est stocké dans un dispositif de stockage (20), et de là, selon les exigences de puissance de refroidissement, est envoyé à la première et/ou à la deuxième phases de refroidissement.

8. Procédé de production d'un alcène selon l'une des revendications précédentes, **caractérisé en ce que** la deuxième phase de refroidissement est mise en oeuvre en amont de la première phase de refroidissement.

9. Procédé de production d'un alcène selon l'une des revendications précédentes, **caractérisé en ce qu'**on obtient en tant que produit supplémentaire (3) de l'hydrogène, et que l'hydrogène produit est, au moins par portions, envoyé dans un réacteur (30) de déshydrogénation pour stabiliser le processus de déshydrogénation.

10. Dispositif pour la production d'un alcène, dans lequel une substance de départ (1) comprenant un hydrocarbure est soumise à une déshydrogénation, et un produit (2) comprenant au moins un alcène est produit, comprenant :
- un réacteur de déshydrogénation (30), pour produire l'alcène à partir de l'hydrocarbure,
- un adsorbeur (40), disposé en aval du réacteur de déshydrogénation (30), pour l'adsorption de l'eau à partir du courant de substance obtenu dans le réacteur de déshydrogénation (30),
- un premier dispositif de refroidissement (14), pour refroidir dans une première phase de refroidissement un courant gazeux (11) provenant d'un adsorbeur (40), grâce au courant gazeux (5) qui comprend la substance de départ, et grâce à un constituant condensé (12) du courant gazeux provenant de l'adsorbeur (40),
- un dispositif de stockage (20) pour stocker le constituant condensé (12) du courant gazeux provenant de l'adsorbeur (40),
- un deuxième dispositif de refroidissement (15) pour refroidir la substance de départ (1) dans une deuxième phase de refroidissement grâce au constituant condensé (12) du courant gazeux provenant de l'adsorbeur (40),
**caractérisé en ce que** le dispositif comprend un dispositif de commande et/ou de régulation (60), à l'aide duquel, d'une manière automatisée, il est possible de faire varier le débit volumique du constituant condensé (12), devant être amené du dispositif de stockage (20) à la première et/ou à la deuxième phase de refroidissement, du courant gazeux provenant de l'adsorbeur (40), en fonction de la température du courant gazeux (13) sortant de la première phase de refroidissement, qui comprend la substance de départ et est envoyé à la déshydrogénation.

11. Dispositif pour la production d'un alcène selon la revendication 10, **caractérisé en ce que** le dispositif de stockage (20) comprend un récipient (23) et/ou au moins un séparateur (21), pour séparer le constituant condensé (12) du courant gazeux provenant de l'adsorbeur (40).

12. Dispositif pour la production d'un alcène selon la revendication 11, **caractérisé en ce que** le dispositif comprend un compartiment (10), thermiquement isolé, dans lequel sont disposés les dispositifs de refroidissement (14, 15, 18), le récipient (23) étant disposé à l'extérieur de ce compartiment thermiquement isolé (10).

13. Dispositif pour la production d'un alcène selon l'une des revendications 10 à 12, **caractérisé en ce que** sur le trajet de l'écoulement du constituant condensé du courant gazeux (12) provenant de l'adsorbeur (40) est disposée une pompe (50) pour refouler dans au moins un dispositif de refroidissement (14, 15, 18) le constituant condensé du courant gazeux (12) provenant de l'adsorbeur.

14. Dispositif pour la production d'un alcène selon l'une des revendications 10 à 13, **caractérisé en ce qu'**au moins l'un des dispositifs de refroidissement (14, 15, 18) comprend un dispositif mélangeur (16, 17) pour mélanger un courant gazeux de recyclage (4) à la substance de départ (1) pour donner un courant gazeux combiné (13).
